# EUROPÄISCHE PATENTANMELDUNG

(11) **EP 2 641 898 A1**
(43) Veröffentlichungstag der Anmeldung: **25.09.2013**
(21) Anmeldenummer: 12160257.7
(22) Anmeldetag: 20.03.2012
(51) Int. Cl.: C07C 319/20, C07C 319/26, C07C 319/28, C07C 323/58

(54) **Verfahren zur Herstellung von Methionin**

(71) Anmelder: Evonik Industries AG, 45128 Essen (DE)
(72) Erfinder: Körfer, Martin, 63796 Kahl (DE); Hasselbach, Hans Joachim, 63571 Gelnhausen (DE); Reichert, Stefan, 60439 Frankfurt (DE); Jakob, Harald, 63594 Hasselroth (DE); Weckbecker, Christoph, 63584 Gründau-Lieblos (DE); Huthmacher, Klaus, 63571 Gelnhausen (DE); Krull, Horst, 63456 Hanau (DE); Drapal, Bernd, 63755 Alzenau (DE); Peter, Rainer, 63829 Krombach (DE)

(57) **Zusammenfassung**

Die Erfindung betrifft ein Verfahren zur Herstellung von D,L-Methionin, bei dem einer durch Hydrolyse von 5-(2-Methylmercaptoethyl)-hydantoin erhaltenen wässrigen Kaliummethioninat-Lösung Kohlendioxid zugeführt wird, um Rohmethionin auszufällen, welches abgetrennt und gereinigt wird, wobei zur Reinigung eine wässrige Lösung des abgetrennten Rohmethionins hergestellt und einer Umkristallisation unterworfen wird, dadurch gekennzeichnet, dass die Lösung, aus der die Umkristallisation erfolgt, Kaliumionen sowie ein Kristallisationsadditiv enthält, wobei das Kristallisationsadditiv ein nichtionisches oder anionisches Tensid, oder eine Mischung aus verschiedenen nichtionischen oder anionischen Tensiden ist, und dass die Umkristallisation durch Einleiten einer 60 bis 110°C heißen Methioninlösung in eine 35 bis 80°C warme Methioninsuspension erfolgt, deren Temperatur niedriger als die der eingeleiteten Lösung ist, wobei die Temperatur der Methioninsuspension während der Zugabe zwischen 35 und 80°C gehalten wird.

## Beschreibung

Die Erfindung betrifft ein Verfahren zur Herstellung von D,L-Methionin mit hoher Schüttdichte, wobei das Methionin durch Umkristallisation gereinigt wird.

L-Methionin ist eine essentielle Aminosäure, die von großer industrieller Bedeutung als Futterzusatzmittel ist. Da D- und L-Methionin von gleichem Nährwert sind, wird üblicherweise das Racemat als Futterzusatzmittel eingesetzt. Die Synthese von D,L-Methionin verläuft ausgehend von Methylmercaptopropionaldehyd und Cyanwasserstoff unter Herstellung des Zwischenprodukts 5-(2-Methylmercaptoethyl)-hydantoin, das durch Hydrolyse zum Methioninat umgesetzt werden kann.

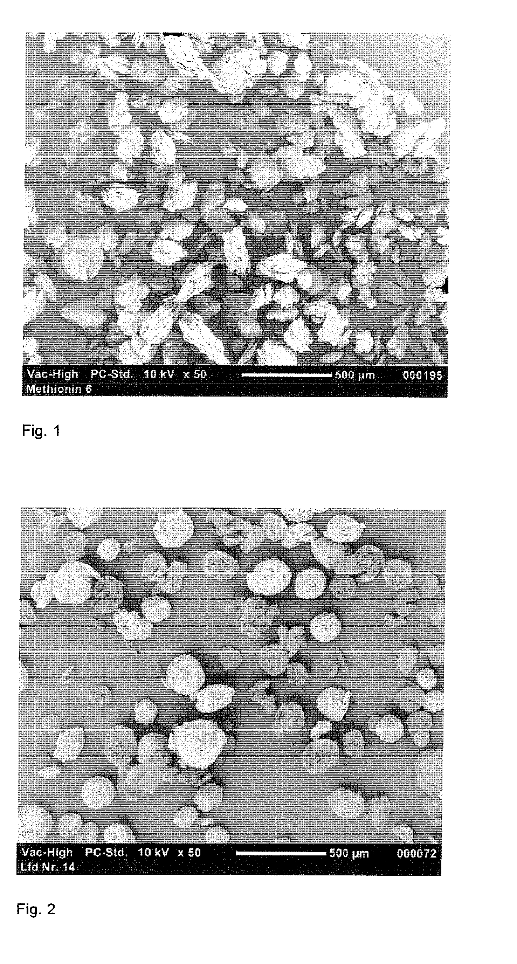

Sowohl für die Hydrolyse des Hydantoins als auch für die anschließende Freisetzung des Methionins aus seinem Salz sind verschiedene Verfahren bekannt. Die vorliegende Erfindung bezieht sich auf die Herstellung von Methionin nach dem sogenannten Kaliumcarbonatverfahren, welches beispielsweise in EP 1 256 571 A1 und DE 19 06 405 A1 beschrieben ist. Dabei wird zunächst 5-(2-Methylmercaptoethyl)-hydantoin in wässriger Lösung mit Kaliumcarbonat unter Freisetzung von Kohlendioxid und Ammoniak zu Kaliummethioninat umgesetzt. Durch Einleiten von Kohlendioxid wird die basische Kaliummethioninat-Lösung neutralisiert und Methionin ausgefällt. Das hierbei gewonnene Rohmethionin fällt jedoch in Form von plättchen- oder schuppenförmigen, schlecht filtrierbaren Kristallen an, die in Figur 1 gezeigt werden.

Zur Beherrschung der Schaumbildung und Verbesserung der Kristallqualität geschieht die Rohmethioninfällung gemäß EP 1 256 571 A1 in Gegenwart eines Entschäumers. Dieses Verfahren hat den Nachteil, dass Methionin in Form von kugelförmigen, jedoch porösen Partikeln, erhalten wird, die in Figur 2 gezeigt werden. Auf Grund seiner porösen Struktur muss das solcherart gewonnene Methionin mit großen Mengen an Wasser gewaschen und unter hohen Energiekosten getrocknet werden, um zu einem marktfähigen Produkt zu gelangen.

Die Zugabe von Additiven während der Rohmethioninfällung kann die Kristallqualität verbessern. Als Additive sind beispielsweise Sorbitanlaurat, Polyvinylalkohol, Hydroxypropylmethylcellulose, Gluten oder Casein aus JP 11158140 und JP 10306071 bekannt. Gemäß dieser Verfahren werden Methioninkristalle mit einem Schüttgewicht von bis zu 770 g/l erhalten. Als nachteilig erweist sich bei diesen Verfahren, dass sie als Batchverfahren oder in lediglich halbkontinuierlicher Verfahrensweise durchgeführt werden.

Es ist ebenfalls bekannt, Reinheit und Schüttgewicht von Methionin durch Umkristallisation von Rohmethionin zu verbessern. JP 2004-292324 offenbart beispielsweise die Umkristallisation von Rohmethionin unter Zugabe von Polyvinylalkohol oder Gluten, wobei Reinmethionin mit einem Schüttgewicht von bis zu 580 g/l erhalten wird. Die Umkristallisation erfolgt durch tröpfchenweise Zugabe einer heißen Methioninlösung zu einer kalten Methioninsuspension, wobei Methionin durch Abkühlen der heißen Lösung ausfällt. Als nachteilig erweist sich wiederum, dass dieses Verfahren nicht kontinuierlich durchgeführt wird.

EP 1 451 139 A1 beschreibt die Umkristallisation von Methionin in Gegenwart von Hydroxyethylcellulose, wobei anfänglich Methioninkristalle mit einem Schüttgewicht von bis zu 620 g/l erhalten werden. Als nachteilig erweist sich in diesem Fall, dass in einem kontinuierlichen Umkristallisationsverfahren durch eine Wiederverwendung des Filtrats zum Auflösen von Rohmethionin eine Anreicherung des kontinuierlich zugegebenen Additivs eintritt und dass eine steigende Additivkonzentration eine Verringerung des Schüttgewichts zur Folge hat. Aus diesem Grund ist Hydroxyethylcellulose für die Verwendung als Kristallisationsadditiv in einem kontinuierlichen Verfahren, bei dem das Filtrat des Reinmethionins zum Lösen von Rohmethionin wiederverwendet wird, unvorteilhaft. Die Wiederverwendung des Filtrats der Umkristallisation ist im großtechnischen Maßstab von entscheidender Bedeutung für die Wirtschaftlichkeit des Verfahrens, da Verluste an gelöstem Methionin vermieden werden und die Erzeugung von Abwasser minimiert wird.

Aufgabe der vorliegenden Erfindung ist es, ein Verfahren zur Herstellung von Methionin bereitzustellen, das die beschriebenen Nachteile vermeidet. Das durch das Verfahren gewonnne Methionin sollte gut filtrierbar sein und ein hohes Schüttgewicht aufweisen. Des Weiteren sollte das Verfahren in kontinuierlicher Verfahrensweise durchführbar sein und insbesondere die negativen Auswirkungen von Anreicherungsprozessen vermeiden.

Zur Lösung dieser Aufgabe stellt die vorliegende Erfindung ein Verfahren zur Herstellung von D,L-Methionin bereit, bei dem einer durch Hydrolyse von 5-(2-Methylmercaptoethyl)-hydantoin erhaltenen wässrigen Kaliummethioninatlösung Kohlendioxid zugeführt wird, um Rohmethioinin auszufällen, welches abgetrennt und gereinigt wird, wobei zur Reinigung eine wässrige Lösung des abgetrennten Rohmethionins hergestellt und einer Umkristallisation unterworfen wird. Dabei enthält die Lösung, aus der die Umkristallisation erfolgt, Kaliumionen sowie ein Kristallisationsadditiv, wobei das Kristallisationsadditiv ein nicht-ionisches oder anionisches Tensid, oder eine Mischung aus verschiedenen nicht-ionischen oder anionischen Tensiden ist. Erfindungsgemäß erfolgt die Umkristallisation durch Einleiten einer 60 bis 110 °C heißen Methioninlösung in eine 35 bis 80 °C warme Methioninsuspension, deren Temperatur niedriger als die der eingeleiteten Lösung ist, wobei die Temperatur der Methioninsuspension während der Zugabe zwischen 35 und 80°C gehalten wird.

Die heiße Methioninlösung wird durch das Einleiten in die vorgelegte, kühlere Methioninsuspension vorzugsweise rasch abgekühlt, wodurch eine Überkonzentration von gelöstem Methionin erzeugt wird und Methionin aus der Lösung ausfällt. Auf diese Weise wird die Präferenz in der Kristallwachstumsraumrichtung durchbrochen und ein isometrischer Kristallhabitus erzielt. Durch diese Verfahrensweise des raschen Abkühlens können jedoch neben den erwünschten, isometrischen Kristallen auch unerwünschte neue, plättchenförmige Kristallkeime entstehen. Diese lassen sich in einer bevorzugten Ausführungsform des erfindungsgemäßen Verfahrens durch eine moderate Temperaturerhöhung um 5-15°C besonders bevorzugt um 6-12°C gegenüber der Mischungstemperatur spezifisch wieder auflösen.

Durch die erfindungsgemäße Kombination aus der Gegenwart von Kaliumionen, der Zugabe von Kristallisationsadditiv und der Temperatursteuerung der Umkristallisation werden grobkörnige, gut filtrierbare Methioninkristalle mit einem Schüttgewicht von über 500 g/l erhalten.

In einer bevorzugten Ausführungsform des Verfahrens ist das Kristallisationsadditiv eine der in Formel 1 bis 3 dargestellten Verbindungen, oder eine Mischung daraus:

R¹-O-SO₃M (Formel 1)

R²-O-(CH₂)ₙ-SO₃M (Formel 2)

R³-(O-C₂H₄)ₙ-O-SO₃M (Formel 3)

wobei n eine ganze Zahl von 1 bis 12, M Natrium oder Kalium und R¹, R² und R³ eine lineare, verzweigte oder cyclische, gesättigte oder ungesättigte C₈ bis C₂₀ Alkylgruppe oder eine Arylgruppe ist.

In einer bevorzugten Ausführungsform der oben genannten Verbindungen ist n = 2 und R¹, R² und R³ sind lineare, gesättigte C₈ bis C₁₈ Alkylgruppen.

In einer weiteren Ausführungsform des Verfahrens ist das Kristallisationsaddtiv ein Sorbitanfettsäureester oder eine Mischung aus verschiedenen Sorbitanfettsäureestern, bevorzugt polyethoxylierte Sorbitanfettsäureester. In einer besonders bevorzugten Ausführungsform ist das Kristallisationsadditiv ein polyethoxyliertes Sorbitantristearat gemäß der Formel 4: 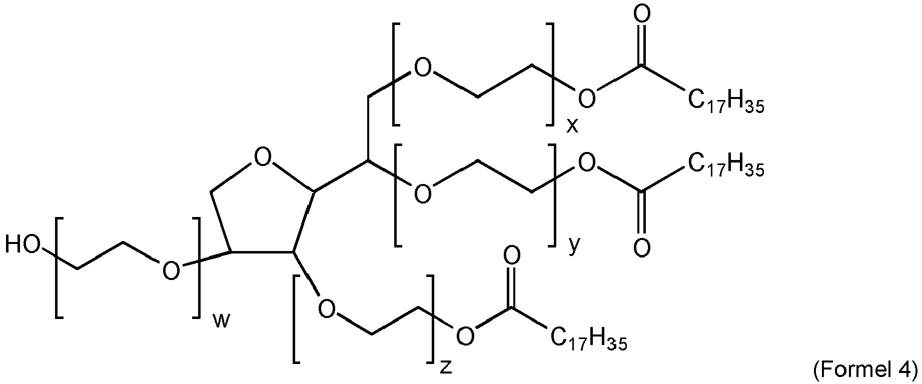 wobei w + x + y+ z =20.

Die Konzentration des Kristallisationsadditivs in der Lösung, aus der die Umkristallisation erfolgt, beträgt vorzugsweise mindestens 50 ppm bezogen auf die Gesamtmasse der Lösung, besonders bevorzugt mindestens 100 ppm, am meisten bevorzugt mindestens 400 ppm. Um eine optimale Dosierung und Verteilung des Kristallisationsadditivs zu erreichen, wird dieses vorzugsweise in Form einer wässrigen Lösung oder Emulsion eingesetzt, wobei die Konzentration des Kristallisationsadditivs in der Lösung oder Emulsion bevorzugt 2 bis 15 Gewichts-% beträgt.

In einer bevorzugten Ausführungsform des erfindungsgemäßen Verfahrens enthält die Lösung, aus der die Umkristallisation erfolgt, zusätzlich einen Entschäumer. Der Entschäumer hat die Funktion den bei der Handhabung der Methioninlösung und - suspension entstehenden und von einigen der oben genannten Kristallisationsadditive verstärkten bzw. verursachten Schaum niederzuschlagen. Darüber hinaus ergibt sich bei einem gleichzeitigen Einsatz von Entschäumer und Kristallisationsadditiven ein synergistischer Effekt bei den erzielten Schüttgewichten des Methionins, wodurch Schüttgewichte von über 600 g/l erreicht, gleichzeitig die negativen Auswirkungen von Anreicherungsprozessen vermieden werden und damit das erfindungsgemäße Verfahren auch in kontinuierlicher Verfahrensweise durchführbar ist.

Der Entschäumer enthält vorzugsweise Silikonöl, wobei bevorzugt ein Silikonöl mit einer kinematischen Viskosität von 0,65 bis 10000 mm²/s (gemessen bei 25°C gemäß DIN 53018), besonders bevorzugt von 90 bis 1500 mm²/s eingesetzt wird. Der Entschäumer kann weiterhin Bestandteile enthalten, die als Emulgatoren wirksam sind, beispielsweise Mischungen polyethoxylierter Fettsäuren und polyethoxylierter Fettalkohole. Der Entschäumer kann ebenfalls Kieselsäure enthalten. In einer bevorzugten Ausführungsform ist der Entschäumer eine wässrige Lösung, die 5 bis 10 Gewichts-% Silikonöl, 0,05 bis 1 Gewichts-% Kieselsäure, 0,5 bis 5 Gewichts-% einer Mischung polyethoxylierter Fettsäuren, sowie 2 bis 7 Gewichts-% einer Mischung polyethoxylierter Fettalkohole enthält. Vorzugsweise wird der Entschäumer in einer Mischung mit dem Kristallisationsadditiv eingesetzt, wobei das Kristallisationsadditiv in einer Konzentration von bevorzugt 2 bis 15 Gewichts-% beigemischt wird. Um eine kontinuierliche, stabile Dosierung des Entschäumers zu erreichen, wird er vorzugsweise vor seinem Einsatz noch weiter mit Wasser verdünnt.

Die Verwendung von Silikonölentschäumern führt dazu, dass in dem nach dem erfindungsgemäßen Verfahren hergestellten Methionin mit einem geeigneten Analysenverfahren (z. B. Röntgenphotoelektronenspektroskopie, kurz XPS) Silicium nachgewiesen werden kann.

Überraschenderweise hat sich gezeigt, dass die Gegenwart von Kaliumionen in der Lösung, aus der die Umkristallisation erfolgt, für den Kristallisationserfolg wichtig ist. Vorzugsweise beträgt die Kaliumionenkonzentration in der Lösung, aus der die Umkristallisation erfolgt, 1 bis 30 g/kg, besonders bevorzugt 2 bis 14 g/kg, am meisten bevorzugt 5 bis 10 g/kg. Das Kalium gelangt bevorzugt mit dem Rohmethionin in die Umkristallisationslösung. Die Kaliumkonzentration kann beispielsweise durch Zufuhr von Waschwasser bei der Rohmethioninfiltration und/oder durch Zufuhr von Frischwasser zu dem zum Lösen des Rohmethionins verwendeten Reinfiltrates und/oder durch Zufuhr von Kalium in das zum Lösen des Rohmethionins verwendete Reinfiltrat eingestellt werden.

Erfindungsgemäß wird das Rohmethionin vor der Umkristallisation in einer wässrigen Lösung gelöst. Dies geschieht bevorzugt durch Erwärmen der Lösung auf eine Temperatur von mindestens 95°C, besonders bevorzugt durch Erwärmen auf Siedetemperatur. Zum Lösen des Rohmethionins können beispielsweise Frischwasser, das Filtrat des Reinmethionins, oder das Kondensat der unten beschriebenen Vakuumkristallisation oder Mischungen hieraus verwendet werden.

Kristallisationsadditiv und der Entschäumer werden erfindungsgemäß der zum Lösen des Rohmethionins verwendeten wässrigen Matrix zugesetzt. In einer möglichen Ausführungsform des Verfahrens werden das Kristallisationsadditiv und der Entschäumer auch der Lösung zugesetzt, aus der das Rohmethionin ausgefällt wird.

Vorzugsweise erfolgt die Umkristallisation durch Einleiten einer 85 bis 110°C heißen Rohmethioninlösung in eine 35 bis 60°C warme Methioninsuspension, wobei die Temperatur der dabei entstehenden Mischung konstant zwischen 35 und 60°C gehalten wird. Dabei liegt das Volumenverhältnis von eingeleiteter Rohmethioninlösung zu vorgelegter Methioninsuspension vorzugsweise im Bereich von 1:1 bis 1:10, besonders bevorzugt von 1:3 bis 1:6.

In einer weiteren bevorzugten Ausführungsform des Verfahrens wird die Umkristallisation zweistufig durchgeführt. Dabei wird in der ersten Umkristallisationsstufe eine 85 bis 110°C heiße Rohmethioninlösung in eine 60 bis 80°C warme Methioninsuspension eingeleitet und die Temperatur der dabei entstehenden Mischung konstant zwischen 60 und 80°C gehalten. Besonders bevorzugt ist es dabei, einen Teil der Methioninsuspension aus der ersten Umkristallisationsstufe zu entnehmen und über einen Umwälzkreislauf wieder in die Umkristallisation zurückzuführen, wobei die Temperatur der Suspension im Umwälzkreislauf um 6 bis 12°C erhöht wird. Die in der ersten Umkristallisationsstufe erhaltene 60 bis 80°C warme Methioninsuspension wird in einer zweiten Umkristallisationsstufe in eine 35 bis 60°C warme Methioninsuspension eingeleitet, wobei die Temperatur der dabei entstehenden Mischung konstant zwischen 35 und 60°C gehalten wird. Das Volumenverhältnis von eingeleiteter zu vorgelegter Methioninsuspension liegt vorzugsweise in einem Bereich von 1:1 bis 1:10, besonders bevorzugt von 1:3 bis 1:6.

Das erfindungsgemäße Verfahren kann neben einer ersten oder einer ersten und zweiten Umkristallisationstufe auch noch weitere Umkristallisationsstufen umfassen.

Bei mehrstufiger Verfahrensweise können alle Stufen parallel mit Rohmethionin beschickt werden bei gleicher Temperaturdifferenz zwischen Rohmethioninlösung und vorgelegter Methioninsuspension. Die mehrstufige Umkristallisation kann auch so durchgeführt werden, dass die Umkristallistionsstufen nacheinander mit der Methioninlösung aus der vorangehenden Stufe beschickt werden, wobei die Temperaturdifferenz zwischen Rohmethionin und Methioninlösung so gewählt wird, dass die Methioninlösung aus einer Umkristallisationsstufe als Rohmethionin der nächsten Umkristallisationsstufe verwendet werden kann. Dies hat den Vorteil der verminderten Bildung unerwünschter plättchenförmiger Kristalle durch zu große Temperaturunterschiede. Selbstverständlich umfasst die mehrstufige Umkristallisation auch Mischformen aus paralleler und nacheinanderfolgender Beschickung der Umkristallisationseinheiten.

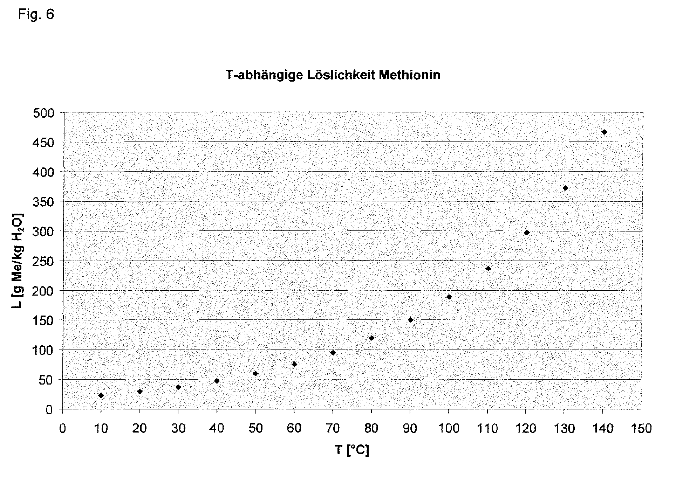

Die bevorzugte Temperaturführung für das erfindungsgemäße Verfahren ergibt sich aus dem in Figur 6 gezeigten temperaturabhängigen Löslichkeitsverhalten von Methionin.

Es ist wirtschaftlich zweckmässig, die Methioninlösungen auf eine Endtemperatur von 30 bis 50°C abzukühlen, da sich so sowohl die Menge an in Lösung verbleibendem Methionin minimieren lässt als auch der Einsatz von teuren Kühlmedien zum weiteren Abkühlen der methioninhaltigen Lösungen vermieden wird.

In einer bevorzugten Ausführungsform des Verfahrens wird die Umkristallisation durch Vakuumkristallisation durchgeführt. Dabei beträgt der Druck in der ersten Umkristallisationsstufe bevorzugt 100 bis 1000 mbar, besonders bevorzugt 150 bis 400 mbar. Falls eine zweistufige Umkristallisation durchgeführt wird, beträgt der Druck in der zweiten Umkristallisationsstufe bevorzugt 35 bis 200 mbar, besonders bevorzugt 35 bis 100 mbar. Vorzugsweise wird das in der Vakuumkristallisation verdampfte Wasser kondensiert und zum Lösen von weiterem Rohmethionin wiederverwendet.

In einer bevorzugten Ausführungsform des Verfahrens wird ein Teil der Methioninsuspension aus der ersten und/oder einer der weiteren Umkristallisationsstufen entnommen und über einen Umwälzkreislauf wieder zurückgeführt. In der ersten Kristallisationsstufe wird die heiße Methioninlösung bevorzugt der umgewälzten kälteren Suspension in einem Volumenverhältnis von 1:3 bis 1:6 zugegeben. Bei dieser raschen Abkühlung wird eine hohe Übersättigung erzeugt durch die einerseits größere Kristalle isometrisch wachsen aber auch neue, kleine, plättchenförmige Kristalle gebildet werden. Die kleinen plättchenförmigen Kristalle werden noch in der Umwälzleitung durch eine Erhöhung der Temperatur um 6 bis 12°C wieder aufgelöst, wobei die isometrischen größeren Kristalle bestehen bleiben.

Die Abtrennung des Reinmethionins von der Mutterlauge der Umkristallisation erfolgt vorzugsweise durch Filtration, beispielsweise Druck- oder Vakuumfiltration, oder mittels Zentrifugen, beispielsweise Schäl-, Schub- oder Siebzentrifugen.

Das erfindungsgemäße Verfahren kann sowohl kontinuierlich als auch nicht- oder halbkontinuierlich durchgeführt werden.

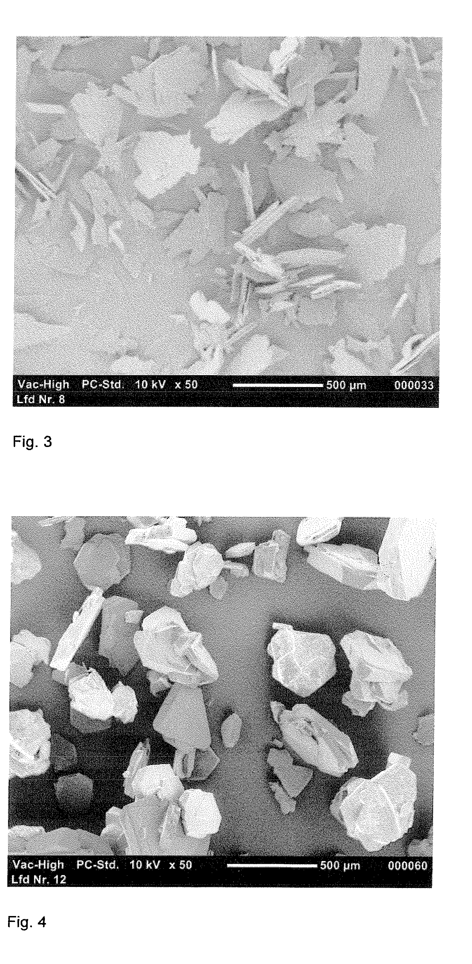

Die beigefügten Figuren 1 bis 4 zeigen elektronenmikroskopische Aufnahmen von kristallinem Methionin. Figur 1 zeigt Rohmethionin, wie es aus der Rohmethioninfällung ohne Zugabe von Kristallisationsadditiven gewonnen wird. Figur 2 zeigt Rohmethionin aus der Rohmethioninfällung unter Zugabe eines Entschäumers gemäß EP 1 256 571 A1. Figur 3 zeigt Methionin wie es ohne Zugabe von Kristallisationsadditiven, ohne die Gegenwart von Kalium durch einfaches Abkühlen erhalten wird. Figur 4 zeigt Reinmethionin, wie es mit dem erfindungsgemäßen Verfahren gewonnen wird.

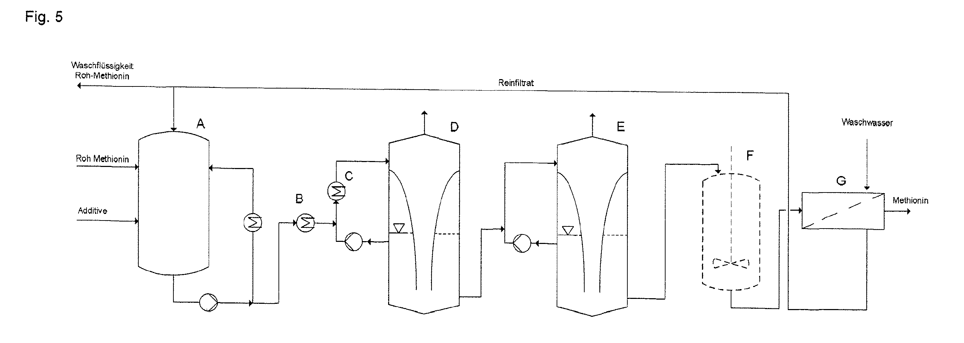

Figur 5 zeigt beispielhaft und schematisch eine Anlage zur Durchführung des erfindungsgemäßen Verfahrens in bevorzugter zweistufiger Umkristallisation. In Behälter A wird Rohmethionin mit einer wässrigen Matrix, die das Filtrat des Reinmethionins enthalten kann, bei einer Temperatur von 90 bis 100°C aufgelöst. Die Temperatur wird über eine Kreislaufpumpe und einen externen Wärmetauscher eingestellt. Der wässrigen Matrix wird kontinuierlich das erfindungsgemäße Kristallisationsadditiv inklusive Entschäumer zugesetzt. Die Methioninlösung wird über einen oder mehrere Wärmetauscher B auf 100 bis 110°C erwärmt und anschließend in den Umwälzkreislauf des ersten Vakuumkristallers D eingeleitet. Die umgewälzte Suspension weist eine Temperatur von 60 bis 70°C auf. Das Verhältnis von eingespeister Menge zu Umlaufmenge liegt im Bereich von 1:3 bis 1:6. Die mittlere Verweilzeit der Mischung im Umwälzkreislauf beträgt 5 bis 15 sec. Die Mischung wird über einen Wärmetauscher C auf 65 bis 75°C erwärmt, wodurch sich feine und insbesondere plättchenförmige Methioninkristalle auf Grund ihrer größeren spezifischen Oberfläche schnell auflösen. Die Mischung gelangt anschließend in den ersten Vakuumkristaller D, in dessen Kopfbereich bei einem Druck von 180 bis 200 mbar Wasserverdampfung und Abkühlung der Mischung eintritt. Dadurch kommt es zur Kristallisation von gelöstem Methionin. Die Methioninkristalle sinken im Vakuumkristaller mit unterschiedlicher Geschwindigkeit ab. Kleine, plättchenförmige Kristalle sinken langsamer als grobe, isometrische Kristalle. Die Suspension zur Umwälzung wird im oberen Bereich des Vakuumkristallers abgenommen, wo sich auf Grund der geringeren Sinkgeschwindigkeit vorwiegend die kleineren, plättchenförmigen Kristalle befinden. Die groben, isometrischen Kristalle werden im unteren Bereich des Vakuumkristallers D abgenommen und in den Umwälzkreislauf des zweiten Vakuumkristallers E eingeleitet. Die hier umgewälzte Suspension weist eine Temperatur von 30 bis 50°C auf. Das Verhältnis von eingespeister Menge zu Umlaufmenge liegt im Bereich von 1:3 bis 1:6. Der Druck im Vakuumkristaller E beträgt 60 bis 80 mbar. Im Vakuumkristaller E wird weiteres Methionin kristallisiert, wodurch sich insbesondere die mittlere Partikelgröße der Methioninkristalle erhöht. Bei Bedarf kann die Methioninsuspension in einen Zwischenbehälter F geleitet werden, um ein Nachfällen von Methionin zu ermöglichen. Das Methionin wird schließlich in einem geeigneten Fest/Flüssigtrennschritt G isoliert, wobei das dabei gewonnene Filtrat bei Bedarf in den Behälter A zurückgeführt werden kann.

Die nachfolgenden Beispiele sollen die Erfindung näher erläutern.

### Beispiele

### Beispiel 1:

### Umkristallisation in Gegenwart eines erfindungsgemäßen Kristallisationsadditivs im Vergleich mit einem bekannten Kristallisationsadditiv

In einem Kolben wurden 60 g Methionin, 305 g Wasser sowie 35 g Rohmethionin-Filtrat vorgelegt und bei einer Temperatur von 40°C im Kreislauf über einen Wärmetauscher umgepumpt. Durch das im Rohmethionin-Filtrat enthaltene Kaliumcarbonat betrug die Kaliumionenkonzentration ca. 7 g/kg. Zu dieser Suspension wurde mit einer Geschwindigkeit von 18 ml/min eine auf 90 °C erhitzte Lösung aus 150 g Methionin in 990,5 ml Wasser und 109,5 g Rohmethionin-Filtrat gegeben, wobei die Temperatur der vorgelegten Suspension bei 40°C gehalten wurde. Nach Zugabe von 650 ml der heißen Lösung wurden 500 ml Suspension entnommen und anschließend weitere 500 ml der heißen Lösung mit einer Geschwindigkeit von 18 ml/min zudosiert. Die entstandene Suspension wurde abgelassen, die Schaummenge bestimmt, das Methionin abfiltriert und mit 300 ml Aceton gewaschen. Nach Trocknung des Methionins wurde das Schüttgewicht bestimmt.

Die Umkristallisationsversuche wurden in Gegenwart der folgenden Zusätze durchgeführt, wobei die angegebene Konzentration durch Zugabe des Additivs in beiden Startlösungen/- suspensionen eingestellt wurde. Die Konzentrationsangabe gibt den Gesamtwirkstoffgehalt des Additivs ohne Wasser bezogen auf die Gesamtmasse der Lösung bzw. Suspension an. Zusatz 1 war eine wässrige Mischung aus Entschäumer und Kristallisationsadditiv gemäß EP 1 451 139 A1, bestehend aus 2 Gewichts-% Hydroxyethylcellulose und 2 Gewichts-% einer polyethoxylierten Fettsäure (C₁₈H₃₇(CO)-O-(CH₂CH₂O)₇-H). Zusatz 2 war eine wässrige Mischung aus einem Kristallisationsadditiv und einer Entschäumerzusammensetzung gemäß der vorliegenden Erfindung, bestehend aus 6,1 Gewichts-% Silikonöl mit einer kinematischen Viskosität von 1000 mm²/s (AK 1000, Wacker-Chemie GmbH), 0,25 Gewichts-% hydrophobierter Kieselsäure (Sipernat D10, Evonik Degussa GmbH), 2,6 Gewichts-% eines polyethoxylierten Fettsäuregemisches (Intrasol® FS 18/90/7, Ashland Deutschland GmbH), 3,7 Gewichts-% eines polyethoxylierten Fettalkoholgemisches (2,35 Gewichts-% Marlipal®, Sasol Germany GmbH, 1,35 Gewichts-% Brij C2, Croda Chemicals Europe) und 5,1 Gewichts-% eines Fettalkoholsulfats (Sulfopon® 1218 G, Oleochemicals) gemäß der Formel:

CₙH₂ₙ₊₁-O-SO₃Na,

wobei n = 12 bis 18.

Die folgende Tabelle zeigt die ermittelten Schaummengen und Methioninschüttgewichte in Abhängigkeit von Art und Konzentration der als Kristallisationszusätze verwendeten Mischungen, wobei der Gesamtwirkstoffgehalt (ohne Wasser) angegeben wird.

| Zusatz | Konzentration (ppm) | Schaummenge (ml) | Schüttgewicht (g/l) |
|---|---|---|---|
| Kein | - | 300 | 507 |
| 1 | 200 | 180 | 614 |
| 1 | 400 | 75 | 626 |
| 1 | 1000 | 10 | 587 |
| 1 | 1200 | 10 | 464 |
| 1 | 2000 | 10 | 410 |
| 1 | 4000 | 5 | 356 |
| 2 | 200 | 40 | 613 |
| 2 | 400 | 5 | 633 |
| 2 | 1000 | 0 | 610 |
| 2 | 1200 | 0 | 651 |
| 2 | 2000 | 0 | 625 |
| 2 | 4000 | 0 | 639 |

Es ist zu beobachten, dass der erfindungsgemäße Kristallisationszusatz bei geringer Konzentration ebenso effektiv das Schüttgewicht verbessert wie der Zusatz gemäß EP 1 451 139 A1 und dass der erfindungsgemäße Zusatz im Gegensatz zu dem Zusatz gemäß EP 1 451 139 A1 seine Wirksamkeit auch bei hoher Konzentration beibehält.

### Beispiel 2:

### Umkristallisation in Gegenwart reiner Entschäumer, reiner Kristallisationsadditive, und Mischungen von Entschäumer und Kristallisationsadditiv

Umkristallisationsversuche entsprechend der Vorschrift aus Beispiel 1 wurden unter Zugabe reiner erfindungsgemäßer Kristallisationsadditive, Mischungen der Kristallisationsadditive mit dem Entschäumer sowie des reinen Entschäumers durchgeführt. Die folgende Tabelle zeigt die dabei ermittelten Schaummengen und Methioninschüttgewichte.

Der reine Entschäumer (Vergleichsbeispiel 1) wurde als wässrige Mischung bestehend aus 6,1 Gewichts-% Silikonöl mit einer kinematischen Viskosität von 1000 mm²/s (AK 1000, Wacker-Chemie GmbH), 0,25 Gewichts-% hydrophobierter Kieselsäure (Sipernat D10, Evonik Degussa GmbH), 2,6 Gewichts-% eines polyethoxylierten Fettsäuregemisches (Intrasol® FS 18/90/7, Ashland Deutschland GmbH), 3,7 Gewichts-% eines polyethoxylierten Fettalkoholgemisches (2,35 Gewichts-% Marlipal®, Sasol Germany GmbH, 1,35 Gewichts-% Brij C2, Croda Chemicals Europe) eingesetzt.

Als reine Kristallisationsadditive wurden die folgenden anioninischen Tenside eingesetzt:
2) CₙH₂ₙ₊₁-O-SO₃Na, mit n = 12 bis 18 (Sulfopon® 1218G, Oleochemicals)
3) CₙH₂ₙ₊₁-O-C₂H₄-SO₃Na, mit n = 8 bis 18 (Hostapon® SCI 85, Clariant)
4) CₙH₂ₙ₊₁-(OC₂H₄)₂-O-SO₃Na, mit n =12 (Disponil® FES 27, Cognis)
5) CₙH₂ₙ₊₁-(OC₂H₄)₁₂-O-SO₃Na, mit n =12 (Disponil® FES 993, Cognis)

Vergleichsbeispiel 6) CₙH₂ₙ₊₁-(OC₂H₄)₃₀-O-SO₃Na, mit n = 12 (Disponil® FES 77, Cognis) Für die Mischungen des Entschäumers mit den Kristallisationsadditiven wurde der oben genannten Mischung jeweils 5,1 Gewichts-% des entsprechenden Kristallisationsadditivs zugegeben und der Wasseranteil um die 5,1 Gewichts-% reduziert:
7) (1) + (2)
8) (1) + (3)
9) (1) + (4)
10) (1) + (5)

### Vergleichsbeispiel 11) (1) + (6)

| Zusatz | Konzentration (ppm) | Schaummenge (ml) | Schüttgewicht (g/l) |
|---|---|---|---|
| Vergleichsbeispiel 1 | 400 | 70 | 474 |
| 2 | 400 | 30-40 | 537 |
| 3 | 400 | 160 | 564 |
| 4 | 400 | >300 | 560 |
| 5 | 400 | >300 | 558 |
| Vergleichsbeispiel 6 | 400 | >400 | 528 |
| 7 | 400 | 5 | 633 |
| 8 | 400 | 5 | 624 |
| 9 | 400 | 20-30 | 613 |
| 10 | 400 | 40 | 581 |
| Vergleichsbeispiel 11 | 400 | 60 | 548 |

Die Ergebnisse zeigen, dass der reine Entschäumer keine Schüttgewichtsverbesserung ergibt (Eintrag 1). Die erfindungsgemäßen Kristallisationsadditive 2 bis 5 verbessern das Schüttgewicht auf Werte >500 g/l, bedingen jedoch in der Mehrzahl der Fälle eine erhöhte Schaumbildung. Die erfindungsgemäßen Kombinationen 7 bis 9 aus Entschäumer und Kristallisationsadditiven führen zu Schüttgewichten >600 g/l, die erfindungsgemäße Kombination 10 zu Schüttgewichten > 500 g/l, ohne dass eine erhöhte Schaumbildung auftritt.

### Beispiel 3:

### Umkristallisation in Gegenwart nichtionischer Tenside

Umkristallisationsversuche entsprechend der Vorschrift aus Beispiel 1 wurden unter Zugabe nichtionischer Tenside durchgeführt. Als nichtionisches Tensid wurde ein polyethoxyliertes Sorbitantmonostearat (Tween™ 65 von Croda) in einer Konzentration von 400 ppm eingesetzt. Dabei wurde ein Methioninschüttgewicht von 616 g/l erzielt.

### Beispiel 4:

### Einfluss der Kaliumionenkonzentration auf das Schüttgewicht von Methionin

Zu einer 40°C warmen Suspension von 20 g Methionin in 180 g Wasser wurden unter Rühren über 2 h 1000 g einer 95°C heißen Lösung aus 100 g Methionin in 900 g Wasser zugetropft, wobei die Temperatur der vorgelegten Suspension bei 40°C gehalten wurde. Die Versuche wurden in Gegenwart von 400 ppm Gesamtwirkstoffgehalt bezogen auf die Gesamtmasse der Lösung/Suspension einer erfindungsgemäßen Mischung eines Kristallisationsadditivs und eines Entschäumers und einer der in der Tabelle angegebenen Kaliumionenkonzentration entsprechenden Menge Kaliumhydrogencarbonat durchgeführt. Die erfindungsgemäße Mischung eines Kristallisationsadditivs und eines Entschäumers bestand aus einer wässrigen Lösung von 6,1 Gewichts-% Silikonöl mit einer kinematischen

Viskosität von 1000 mm²/s (AK 1000, Wacker-Chemie GmbH), 0,25 Gewichts-% hydrophobierter Kieselsäure (Sipernat D10, Evonik Degussa GmbH), 2,6 Gewichts-% eines polyethoxylierten Fettsäuregemisches (Intrasol® FS 18/90/7, Ashland Deutschland GmbH), 3,7 Gewichts-% eines polyethoxylierten Fettalkoholgemisches (2,35 Gewichts-% Marlipal®, Sasol Germany GmbH, 1,35 Gewichts-% Brij C2, Croda Chemicals Europe) und 5,1 Gewichts-% eines Fettalkoholsulfats (Sulfopon® 1218 G, oleochemicals) gemäß der Formel:

CₙH₂ₙ₊₁-O-SO₃Na,

wobei n = 12 bis 18. Die Konzentration des reinen Kristallisationsadditivs betrug 117 ppm.

Das Schüttgewicht des ausgefällten Methionins wurde nach Filtration und Trocknung bestimmt.

| K⁺-Konzentration (g/l) | Schüttgewicht (g/l) |
|---|---|
| 0 | 160 |
| 2 | 560 |
| 4 | 590 |
| 8 | 570 |
| 10 | 570 |
| 12 | 560 |
| 14 | 540 |

Die Zugabe von Kaliumionen führt demnach zu einer Verbesserung des Schüttgewichts auch bei geringer Konzentration des als Kristallisationsadditivs eingesetzten Fettalkoholsulfats.

## Patentansprüche

1. Verfahren zur Herstellung von D,L-Methionin, bei dem einer durch Hydrolyse von 5-(2-Methylmercaptoethyl)-hydantoin erhaltenen wässrigen Kaliummethioninat-Lösung Kohlendioxid zugeführt wird, um Rohmethionin auszufällen, welches abgetrennt und gereinigt wird, wobei zur Reinigung eine wässrige Lösung des abgetrennten Rohmethionins hergestellt und einer Umkristallisation unterworfen wird, **dadurch gekennzeichnet, dass** die Lösung, aus der die Umkristallisation erfolgt, Kaliumionen sowie ein Kristallisationsadditiv enthält, wobei das Kristallisationsadditiv ein nichtionisches oder anionisches Tensid, oder eine Mischung aus verschiedenen nichtionischen oder anionischen Tensiden ist, und dass die Umkristallisation durch Einleiten einer 60 bis 110°C heißen Methioninlösung in eine 35 bis 80°C warme Methioninsuspension erfolgt, deren Temperatur niedriger als die der eingeleiteten Lösung ist, wobei die Temperatur der Methioninsuspension während der Zugabe zwischen 35 und 80°C gehalten wird.

2. Verfahren nach Anspruch 1, **dadurch gekennzeichnet, dass** das Kristallisationsadditiv eine der in Formel 1 bis 3 dargestellten Verbindungen, oder eine Mischung daraus ist:
R¹-O-SO₃M (Formel 1)
R²-O-(CH₂)ₙ-SO₃M (Formel 2)
R³-(OC₂H₄)ₙ-O-SO₃M (Formel 3)
wobei n eine ganze Zahl von 1 bis 12, M Natrium oder Kalium und R¹, R² und R³ eine lineare, verzweigte oder zyklische, gesättigte oder ungesättigte C₈ bis C₂₀ Alkylgruppe oder eine Arylgruppe ist.

3. Verfahren nach Anspruch 2, **dadurch gekennzeichnet, dass** das n gleich 2 und R¹, R² und R³ lineare, gesättigte C₈ bis C₁₈ Alkylgruppen sind.

4. Verfahren nach Anspruch 1, **dadurch gekennzeichnet, dass** das Kristallisationsadditiv ein Sorbitanfettsäureester oder eine Mischung aus verschiedenen Sorbitanfettsäureestern ist.

5. Verfahren nach einem der Ansprüche 1 bis 4, **dadurch gekennzeichnet, dass** die Konzentration des Kristallisationsadditivs in der Lösung, aus der die Umkristallisation erfolgt, mindestens 50 ppm bezogen auf die Gesamtmasse der Lösung bzw. Suspension beträgt.

6. Verfahren nach einem der Ansprüche 1 bis 5, **dadurch gekennzeichnet, dass** die Lösung, aus der die Umkristallisation erfolgt, zusätzlich einen Entschäumer enthält.

7. Verfahren nach Anspruch 6, **dadurch gekennzeichnet, dass** der Entschäumer Silikonöl enthält.

8. Verfahren nach einem der Ansprüche 1 bis 7, **dadurch gekennzeichnet, dass** die Kaliumionenkonzentration in der Lösung, aus der die Umkristallisation erfolgt, 1 bis 30 g/kg beträgt.

9. Verfahren nach einem der Ansprüche 1 bis 7, **dadurch gekennzeichnet, dass** die Kaliumionenkonzentration in der Lösung, aus der die Umkristallisation erfolgt, 5 bis 10 g/kg beträgt.

10. Verfahren nach einem der Ansprüche 1 bis 9, **dadurch gekennzeichnet, dass** die Umkristallisation durch Einleiten einer 85 bis 110°C heißen Rohmethioninlösung in eine 35 bis 60°C warme Methioninsuspension erfolgt, wobei die Temperatur der dabei entstehenden Mischung konstant zwischen 35 und 60°C gehalten wird.

11. Verfahren nach einem der Ansprüche 1 bis 9, **dadurch gekennzeichnet, dass** die Umkristallisation zweistufig durchgeführt wird, wobei in der ersten Umkristallisationsstufe eine 85 bis 110°C heiße Rohmethioninlösung in eine 60 bis 80°C warme Methioninsuspension eingeleitet und die Temperatur der dabei entstehenden Mischung konstant zwischen 60 und 80°C gehalten wird und wobei die in der ersten Umkristallisationsstufe erhaltene 60 bis 80°C warme Methioninsuspension in einer zweiten Umkristallisationsstufe in eine 35 bis 60°C warme Methioninsuspension eingeleitet wird, wobei die Temperatur der dabei entstehenden Mischung konstant zwischen 35 und 60°C gehalten wird.

12. Verfahren nach einem der Ansprüche 1 bis 11, **dadurch gekennzeichnet, dass** die Umkristallisation durch Vakuumkristallisation erfolgt, wobei der Druck in der ersten Umkristallisationsstufe 100 bis 1000 mbar und, falls eine zweistufige Umkristallisation durchgeführt wird, in der zweiten Umkristallisationsstufe 35 bis 200 mbar beträgt.

13. Verfahren nach einem der Ansprüche 1 bis 12, **dadurch gekennzeichnet, dass** ein Teil der Methioninsuspension aus der ersten oder einer der weiteren Umkristallisationsstufen entnommen und über einen Umwälzkreislauf wieder zurückgeführt wird, wobei die Temperatur der Suspension im Umwälzkreislauf um 6 bis 12°C erhöht wird.
